# EUROPEAN PATENT APPLICATION

(11) **EP 0 527 651 A1**
(43) Date of publication of application: **17.02.1993**
(21) Application number: 92307417.3
(22) Date of filing: 13.08.1992
(51) Int. Cl.: A61B 8/00, G10K 11/02

(54) **Acoustic standoff for ultrasound scanhead**

(30) Priority: 14.08.1991 US 745040
(71) Applicant: ADVANCED TECHNOLOGY LABORATORIES, INC., Bothell, Washington 98041 (US)
(72) Inventor: Oaks, Frank Bentley, Kent, WA 98031 (US); Mumford, Wayne H., Bothell, WA 98012 (US)
(74) Representative: Mercer, Christopher Paul

(57) **Abstract**

An acoustic standoff is provided for an ultrasonic scanhead which readily attaches to and detaches from an ultrasonic scanhead. The standoff includes a retention clip which snaps onto the scanhead and defines a volumetric region in front of the scanhead acoustic window. A body of acoustically transmissive material which is preferably disposable is located in the volumetric region. This integral configuration of scanhead and disposable standoff material advantageously allows a clinician to manipulate both the scanhead and its standoff with one hand during performance of an ultrasonic examination. Also disclosed is a convenient kit for the retention clip and disposable blocks of standoff material which enables standoff material of the proper dimensions and acoustic characteristics for the clip to be cast and formed directly in compartments of the kit.

## Description

This invention relates to apparatus for ultrasonically examining superficial structure of the body and, in particular, to acoustic standoff apparatus for elevating a scanhead above such body structure during ultrasonic scanning.

Scanheads for medical ultrasonic examination customarily include an ultrasonic transducer mounted on or immediately adjacent to the surface of the scanhead unit for the transmission of ultrasonic energy into the body and the reception of echo energy returned by structure within the body. The transducer may comprise a single piezoelectric element, or a plurality of adjacently disposed elements such as an array transducer. Generally the transducer is covered with a material which shields and protects the transducer elements but is highly transmissive of ultrasonic energy, known as an acoustic window through which the ultrasonic energy passes. In use of the scanhead the skin of the patient is coated with a viscous ultrasonic couplant gel to efficiently couple the ultrasonic energy between the acoustic window of the scanhead and the skin of the patient, and the acoustic window is pressed against the couplant-coated skin. Thus, the transducer itself is generally located only a few millimeters above the skin line during scanning, and ideally ultrasonic energy passes into and returns from the body of the patient through the couplant and acoustic window with no significant attenuation or distortion.

It is generally desirable to focus the ultrasonic energy being transmitted into the body at a particular depth within the body. This would be the depth, for instance, of a particular organ or body structure which is the subject of the ultrasonic examination. Focussing may be accomplished a number of ways, such as by the geometry of the transducer or the timed actuation of individual elements of an array transducer. Thus, while the beam of transmitted ultrasonic energy may be as wide as the transducer itself when it emanates from the acoustic window of the scanhead and enters the body, it can be narrowed to a more narrowly focussed beam at some further depth within the body. Such focussing provides clearer, more highly defined acoustic information about the body structure being scanned.

However, when the scanhead is being used for many peripheral vascular applications the body structure being examined is frequently located just beneath the skin surface. Such applications include ultrasonic studies of saphenous veins, dialysis shunts and digital arteries, and superficial structures such a shoulders, testicles, thyroid, and subcutaneous tissue. In these applications the focal range of the scanhead, whether geometrically or electronically determined, is at a greater depth from the skin surface than the structure being examined. Accordingly it is desirable to provide a means whereby the focal range can be relocated to the subcutaneous or superficial locations of these structures.

The prior art contains several solutions to this problem involving the use of some medium transmissive of ultrasound between the acoustic window of the scanhead and the surface of the skin. Devices which have been used for this purpose include water bags, filled IV bags, and commercially available acoustic standoff pads which are placed on the skin. The acoustic window of the scanhead is pressed against the device and the thickness of the device effectively elevates the scanhead above the skin surface so that the focal range of the scanhead is just below the skin surface. Another approach that has been employed is to layer a thickness of couplant gel on the skin, then hold the scanhead so that the acoustic window makes minimal contact with the gel surface. All of these approaches require the use of some device or substance separate from the scanhead which must be held or controlled to provide the desired offset between the scanhead and the skin surface. Furthermore, most of these approaches are at best imprecise in the degree of offset provided, requiring at time some considerable experimentation by the user before the desired offset is achieved. In addition some of these approaches undesirably introduce surface artifacts into the received ultrasonic signals such as those caused by the liquid-bag interface of a water or IV bag.

In accordance with the principles of the present invention an acoustic standoff arrangement is provided which integrally attaches to an ultrasonic scanhead. The arrangement includes a retention clip which readily and easily attaches to the scanhead. The retention clip defines a volumetric region in front of the acoustic window of the scanhead in which a body of acoustically transmissive material is located. The retention clip thus retains the acoustically transmissive material in place in front of and in contact with the acoustic window and also defines a predetermined standoff distance between the acoustic window of the scanhead and the retention clip's own acoustic window. The rigidity of the retention clip enables the acoustic window of the clip to be pressed against the skin of the patient during ultrasonic scanning without deformation of the acoustically transmissive material beyond predetermined limits, and the patient is scanned by ultrasonic energy transmission through the material with the focal region of scanhead located just beneath the skin line of the patient.

In the drawings:
FIGURE 1 is a perspective view of an ultrasonic scanhead and an acoustic standoff arrangement constructed in accordance with the principles of the present invention;
FIGURE 2 is an end view of an ultrasonic scanhead with an attached acoustic standoff arrangement of the present invention;
FIGURE 3 is a side view of an ultrasonic scanhead with an attached acoustic standoff arrangement of the present invention in which the retention clip is shown in cross- section; and
FIGURE 4 is a perspective view of an acoustic standoff kit enclosing the elements of an acoustic standoff arrangement of the present invention.

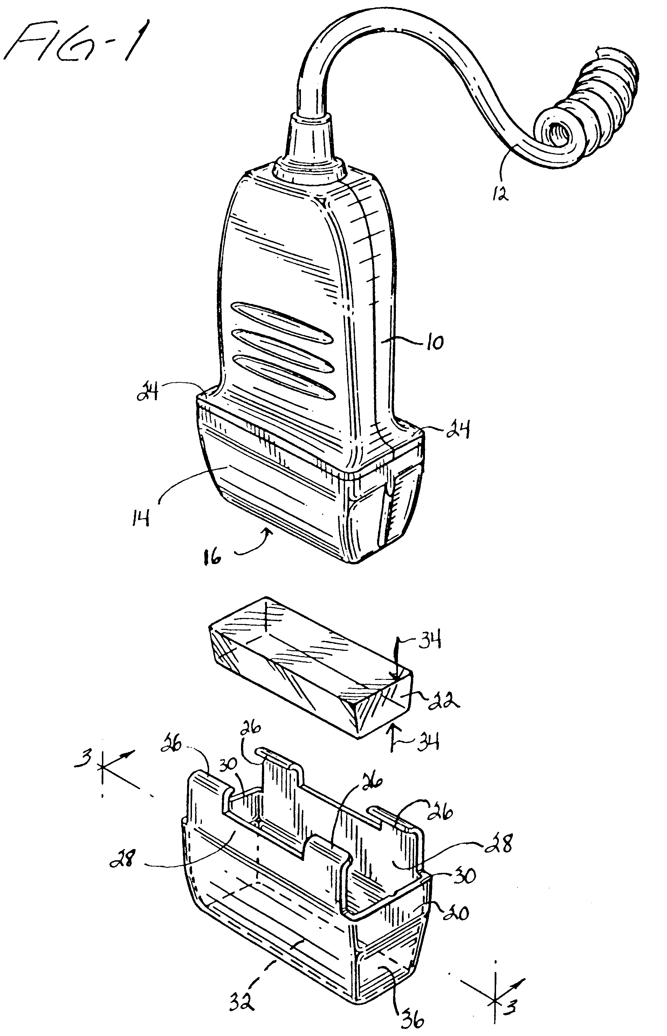

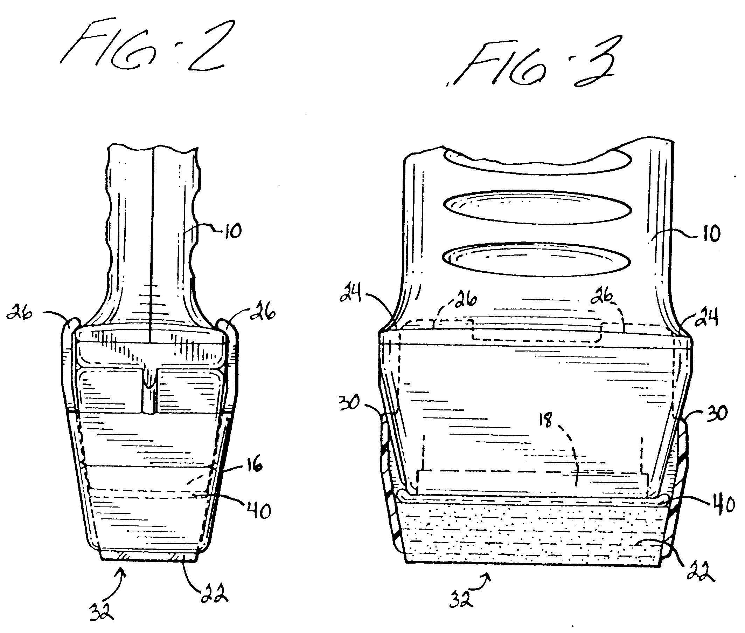

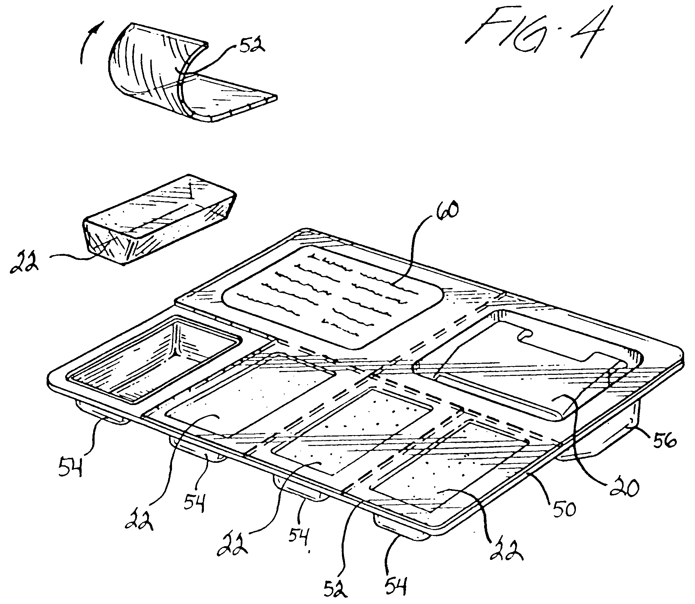

Referring first to FIGURE 1, a typical ultrasonic scanhead 10 is shown. The scanhead includes a plastic housing which contains the electronic components of the scanhead, including the transducer element or elements 18 (see FIGURE 3) and electrical connections between a cable and the transducer elements. Extending from the proximal end of the housing is a cable 12 through which electrical signal to and from the tranducer elements are transmitted. The remote end of the cable 12 (not shown) terminates in a connector suitable for connection to a mainframe ultrasonic diagnostic instrument. The instrument develops timed transducer energization signals which are transmitted through the cable to cause the elements to transmit ultrasonic wave energy. Returning echo signals received by the transducer elements are converted by the elements to electrical signals then transmitted through the cable to the mainframe instrument where they are processed to provide diagnostic information about the body structure of a patient. This information may be in the form of an ultrasonic image of the internal structure of the patient, the velocity of blood flow, and the like.

The distal end 14 of the scanhead encloses transducer elements in close proximity to the distal end or face of the scanhead indicated at 16. The elements are insulated and protected by an acoustic window of silicone rubber at the face 16 of the scanhead 10. During ultrasonic scanning of depths of the body which do not require an acoustic standoff the face 16 of the scanhead is pressed against the skin of the patient, with a coating of ultrasonic couplant providing reliable acoustic transmission between the scanhead and the body.

Shown at the bottom of FIGURE 1 is a retention clip 20. The retention clip is preferably a molded one-piece unit and made of a rigid but somewhat flexible plastic material such as a polycarbonate material. The retention clip 20 defines a volumetric region in which a body 22 of acoustically transmissive material may be located. At the proximal end of the clip 20 are four bent tabs 26 which extend from the flexible proximal sides 28 of the clip. The retention clip is removably attached to the scanhead 10 by insertion of the distal end 14 of the scanhead into the proximal end of the clip. As the scanhead is inserted into the clip the sides of the scanhead contact the ends of the tabs 26 and deflect the proximal sides 28 of the clip outward until the tabs snap over a shoulder 14 of the scanhead. At this time the edges 30 at the ends of the clip are in contact with the lateral sides of the scanhead which, in combination with the locking action of the tabs, maintain the retention clip fixedly attached to the scanhead. FIGURE 3 also illustrates this retentive arrangement.

Before the retention clip 20 is snapped onto the scanhead 10 the body 22 of acoustically transmissive material is placed inside the clip. The body 20 may be made of any material which exhibits the desired acoustic properties for the transfer of ultrasonic energy between the acoustic window of the scanhead and the body with minimal attenuation, similar acoustic velocity, and good impedance-matching properties. Soft rubber and polymeric materials may be used, with a preferred material being a gelatinous material such as a hydrogel. Gelatinous materials are preferred because of their high water content which closely matches the acoustic characteristics of tissue. Hydrogels, in which a hydrophilic carrier entraps a high concentration of water in a shape-retaining gelatinous structure, favorably affords low attenuation of ultrasonic energy, desirable acoustic velocity, and excellent impedance- matching properties, together with the ability to retain its shape in the retention clip for the duration of an examination procedure. With these properties the hydrogel will pass ultrasonic energy between the scanhead and the body with minimal loss of energy and substantially no distortion. The hydrogel may be cast in a rectangular block which is readily retained by the tapered interior of the distal end 36 of the retention clip. The block 22 of hydrogel which, in the illustrated embodiment, has a thickness of approximately 15 mm as indicated by arrows 34 in FIGURE 1, is simply dropped into the retention clip. The rectangular block of hydrogel conforms to the tapered interior surfaces of the distal end of the retention clip as shown in FIGURES 2 and 3, which retains the block in place in the distal end of the clip.

A commercially available material which may be used for the acoustically transmissive body 22 is an aqueous gel pad known as the Aquaflex^{(R)} Ultasound Gel Pad, available from Parker Laboratories, Inc. of Orange, New Jersey, USA. These gel pads have desirable acoustic properties, are bacteriostatic and hypoallergenic. A body 22 for the retention clip 20 may be easily cut in the desired shape from one of these pads and placed inside the volumetric region of the clip. Alternatively, the open acoustic window 32 of the clip 20 can be pressed firmly against the pad in the manner of a cookie cutter, thereby simultaneously cutting and forcing a section of the pad into the volumetric region of the clip.

A coating 40 of conventional ultrasonic couplant is spread along the upper surface of the body 22 or, preferably, on the face 16 of the scanhead to provide good acoustic coupling between the acoustic window of the scanhead and the body of acoustically transmissive material. When the retention clip is then snapped onto the scanhead as shown in FIGURES 2 and 3, the face of the scanhead contacts the body 22 through the ultrasonic couplant medium which depresses the body to slightly extend through the open acoustic window 32 at the distal end of the clip. FIGURES 2 and 3 also illustrate the excellent acoustic coupling which occurs by virtue of the contact between the face 16 of the scanhead, the couplant 40, and the acoustically transmissive body 22.

In use, the scanhead with the attached retention clip is pressed against the couplant-coated skin of the patient. This places the extended body 22 in good acoustic contact with the patient. Depending upon the amount of pressure applied by the user, the acoustically transmissive material may be compressed back into the retention clip. However the rigid surrounding sides of retention clip prevent compression of the thickness of the body to less than the desired standoff spacing of 15 mm or other predetermined distance. Thus, tolerances in the manufacture of the body 22 need only be loosely maintained, as the internal tapering and spacing dimensions of the retention clip cause the body to be compressed to its desired retentive shape and standoff spacing.

The internal tapering and spacing dimensions of the retention clip provide advantage over many prior art standoffs in the reduction of reverberation effects. Referring to FIGURES 2 and 3, as the ultrasonic beam emanates from the transducer 18 it begins to diverge. With some of the elongate, narrow standoffs of the prior art this divergence can cause the lateral extremities of the beam to impinge upon surfaces of the standoff and be reflected back to the transducer, interfering with the reception of the desired acoustic echoes from the subject under examination. However, the illustrated embodiment, with its wide acoustic window 32 and low profile, such as the above-mentioned standoff spacing of 15 mm, reduces the possibility of reverberation effects from the interior surfaces of the retention clip 20. In addition to the ability to scan a patient with a mobile scanhead and standoff that can be held and controlled by a single hand of the clinician, the present invention provides this capability with artifact-free performance.

When the ultrasonic examination is finished the retention clip easily unsnaps from the scanhead. The hydrogel block or other body of acoustically transmissive material is then disposed of and the retention clip and scanhead cleaned of couplant in preparation for another examination.

FIGURE 4 illustrates a kit which provides an ultrasonic system user with a retention clip and sufficient hydrogel blocks for four examinations. The kit includes a molded or formed tray 50 made of polystyrene or comparable material. The tray 50 is formed to have four hydrogel block compartments 54 and a larger compartment 56 for a retention clip. The hydrogel is preferably cast directly in the four compartments 54 which give the blocks their desired shape and dimensions. With the retention clip 20 placed in its compartment 56, the top of the tray 50 is sealed by a cover 52 to hold the kit components in place and to prevent dessication of the hydrogel. The cover 52 may be a single sheet of polymeric material which overlies the entire top of the tray 50 as shown in FIGURE 4, in which case the cover may be appropriately scored as shown to enable separate sections 52′ of the cover to be peeled away without exposing hydrogel blocks to be used at a later time. Alternatively, individual covers can be formed to engage the openings of the five compartments and thereby seal the compartments individually. Space is also provided on the tray 50 or cover 52 for a label 60 to provide the user with information concerning the use and operation of the standoff kit. When the retention clip 20 is formed as a low cost, one- piece molded unit as described herein, it also may be disposed of after the four hydrogel blocks of the kit have been consumed and a new kit ordered.

It is seen that the acoustic standoff arrangement of the present invention provides a precisely specified standoff spacing in an easy to use kit form. The standoff quickly snaps onto and off of the scanhead with minimal inconvenience to the user. Importantly, the attached standoff does not need to be held in place by the user and the scanhead can be held and manipulated virtually as though the standoff were not present. The preferred hydrogel block is inexpensive, exhibits the desired qualities of low acoustic attenuation and good impedance matching properties, and is disposable. When the ultrasonic examination is finished, the standoff arrangement is easily detached from the scanhead and cleaned in preparation for the next examination for which it is required. Finally, the provision of the standoff elements in kit form, including the hydrogel blocks, provides conveniences of ordering, storage, and availability for the user.

## Claims

1. An acoustic standoff for an ultrasonic scanhead having an acoustic window through which ultrasonic energy is transmitted or received comprising:
a retention member suitable for attachment to an ultrasonic scanhead and defining a volumetric region between said scanhead acoustic window and an acoustic window of said retention member when so attached; and
a volumetric member which is transmissive of ultrasonic energy between said scanhead acoustic window and a subject, said volumetric member being dimensioned to be located within said volumetric region of said retention member when said retention member is attached to an ultrasonic scanhead.

2. The acoustic standoff of Claim 1, wherein said volumetric member is made of a deformable material exhibiting relatively low acoustic attenuation and favorable acoustic impedance.

3. The acoustic standoff of Claim 2, wherein said volumetric member is made of a material having a high water content.

4. The acoustic standoff of Claim 3, wherein said volumetric member is made of a gelatinous material.

5. The acoustic standoff of Claim 1, wherein said retention member includes means for engaging said scanhead and means for connecting said retention member to said scanhead in a positionally fixed yet removable manner.

6. The acoustic standoff of Claim 3, wherein said retention member is a one-piece retention clip.

7. The acoustic standoff of Claim 1, further including a coating of ultrasonic couplant between said volumetric member and said scanhead acoustic window.

8. The acoustic standoff of Claim 7, wherein said retention member acoustic window comprises an opening at a side of said retention member opposing said scanhead acoustic window, whereby a surface of said volumetric member is located approximately within said retention member acoustic window during use of said standoff.

9. The acoustic standoff of Claim 8, wherein an internal dimension of said volumetric region is less than an external dimension of said volumetric member, whereby said volumetric member is retained inside said retention member.

10. An acoustic standoff kit for an ultrasonic scanhead having an acoustic window through which ultrasonic energy is transmitted or received comprising:
a retention member suitable for attachment to an ultrasonic scanhead and defining a volumetric region between said scanhead acoustic window and an acoustic window of said retention member when so attached; and
a plurality of disposable volumetric members intended for use in a single ultrasonic examination, said members being transmissive of ultrasonic energy between said scanhead acoustic window and a subject, said volumetric members each being dimensioned to be located within said volumetric region of said retention member when said retention member is attached to an ultrasonic scanhead; and
means for packaging said retention member and said plurality of disposable volumetric members.

11. The acoustic standoff kit of Claim 10, wherein said packaging means includes a plurality of separate compartments for containing individual ones of said volumetric members, wherein said separate compartments can be individually opened.

12. The acoustic standoff kit of Claim 11, wherein said volumetric members are formed of substantially liquid material poured into said separate compartments, whereby the dimensions of said compartments provide said volumetric members with their desired shapes.

13. The acoustic standoff kit of Claim 12, wherein said separate compartments are sealed to minimize dessication of said volumetric members.
